# EUROPEAN PATENT APPLICATION

(11) **EP 1 813 309 A1**
(43) Date of publication of application: **01.08.2007**
(21) Application number: 05799102.8
(22) Date of filing: 25.10.2005
(51) Int. Cl.: A61P 43/00

(54) **ABNORMAL PROTEIN REMOVING COMPOSITION**

(30) Priority: 28.10.2004 JP 2004314871
(71) Applicant: Fancl Corporation, Kanagawa 231-0806 (JP)
(72) Inventor: ONO, Erika, Yokohama-shi, Kanagawa 244-0806 (JP); MIYATA, Satoshi, Yokohama-shi, Kanagawa 244-0806 (JP)
(74) Representative: Vossius & Partner
(86) International application number: PCT/JP2005/019596
(87) International publication number: WO 2006/046557

(57) **Abstract**

Provide an effective composition for removing abnormal protein. Also, a composition for removing abnormal protein whose main ingredient is soyasaponin B group, which is used to provide a composition, agent or food contributing to the prevention or treatment of diseases caused by proteolysis abnormality, among others.

## Description

### BACKGROUND OF THE INVENTION

### Technical Field

The present invention relates to a composition for removing abnormal protein, as well as a composition for preventing diseases involving abnormal protein accumulation.

### Description of the Related Art

Abnormal protein accumulates with age, and it has been shown that proteolysis abnormality of accumulated protein in our body is associated with a number of diseases and disorders (Alzheimer's disease, Parkinson's disease, dementia with Lewy bodies, triplet repeat disease, amyotrophic lateral sclerosis, cataract, arteriosclerosis, diabetic nephropathy, skin aging due to light, wrinkles, etc.) (Non-Patent Literature: BIO Clinica, Vol. 11, No. 5, 1996). Accumulation of abnormal protein may also accelerate in young people suffering from chronic production of a large amount of active oxygen in the body due to excessive stress, exposure to UV ray, etc. Currently, prevention and treatment of diseases caused by proteolysis abnormality is presenting a significant challenge. For many years, research has been conducted to prevent oxygen modification of protein and thereby prevent accumulation of abnormal protein in the body. To be specific, what this research aims to accomplish is to eliminate, through intake of antioxidants, active oxygen that has accumulated due to stress to a level where it can no longer be processed by the natural defense mechanism of our body, thereby suppressing the oxidization of protein. Representative antioxidants include tocophenols, carotinoids, and flavonoids contained in various types of plants.

However, while intake of antioxidants contributes to the elimination of active oxygen generated in the body, it does not contribute at all to the removal of abnormal protein already accumulated in the body. To prevent and treat various diseases associated with abnormal protein that accumulates in our body as we age, abnormal protein must be removed.

On the other hand, the inventors focused on the action of soyasaponin and conducted a series of studies to explore this substance, through which the inventors already presented a number of functions provided by soyasaponin, including a function to remove abnormal protein (Patent Literature 1: Japanese Patent Laid-open No. 2002-179592; Patent Literature 2: Japanese Patent Laid-open No. 2004-115438) and a function to prevent or repair damage caused by UV ray (Patent Literature 3: Japanese Patent Laid-open No. 2004-131431).
Also, the inventors looked for other components that would eliminate abnormal protein and found that in addition to soyasaponin, kale would also provide this action (Patent Literature 4: Japanese Patent Laid-open No. 2004-91398).
It was found that when the aforementioned soyasaponin was used in the prevention and treatment of various diseases associated with abnormal protein that occur with advancing age, a large quantity must be ingested as a normal intake, and accordingly the inventors worked to develop and find a different component offering greater effects at smaller quantities.

Patent Literature 1: Japanese Patent Laid-open No. 2002-179592
Patent Literature 2: Japanese Patent Laid-open No. 2004-115438
Patent Literature 3: Japanese Patent Laid-open No. 2004-131431
Patent Literature 4: Japanese Patent Laid-open No. 2004-91398

### SUMMARY OF THE INVENTION

### Problems to Be Solved by the Invention

The object of the present invention is to focus on soyasaponin to specify and provide an effective composition for removing abnormal protein, and also provide a composition, agent or food that contributes to the prevention and treatment of diseases caused by proteolysis abnormality, among others.

### Means for Solving the Problems

To achieve the aforementioned object, the inventors used fractions of soyasaponin to find components that would remove abnormal protein. As a result, the inventors found the intended effects in highly pure soyasaponin B group and completed the present invention.
The key constitutions of the present invention are as follows:

(1) A composition for removing abnormal protein, characterized by having soyasaponin B group as a main ingredient.
(2) A composition for removing abnormal protein according to (1), characterized in that soyasaponin B group is a soybean extract containing 40 to 100 parts by weight of soyasaponin B group relative to 100 parts by weight of the soybean extract.
(3) A composition for removing abnormal protein according to (1) or (2), characterized in that soyasaponin B group is soyasaponin I, II, III, IV or V or acetylated form thereof, and soyasaponin A group is soyasaponin A1, A2, A3, A4, A5, A6, Ac or Ad or acetylated form or mixture thereof.
(4) A composition for removing abnormal protein according to any one of (1) to (3), characterized by containing one or more of collagen, gelatin, collagen hydrolyzate and gelatin hydrolyzate, and/or silymarin.
(5) A composition for preventing or repairing UV damage, containing a composition for removing abnormal protein according to any one of (1) to (4).
(6) An anti-aging food containing a composition for removing abnormal protein according to any one of (1) to (4) or a composition for preventing or repairing UV damage according to (5).
(7) A food for suppressing dull complexion or wrinkles or providing moisture-keeping effect, containing a composition for removing abnormal protein according to any one of (1) to (4) or a composition for preventing or repairing UV damage according to (5).
(8) An anti-aging cosmetic containing a composition for removing abnormal protein according to any one of (1) to (4) or a composition for preventing or repairing UV damage according to (5).
(9) A cosmetic for suppressing dull complexion or wrinkles or providing moisture-keeping effect, containing a composition for removing abnormal protein according to any one of (1) to (4) or a composition for preventing or repairing UV damage according to (5).
(10) An anti-aging feed or animal drug containing a composition for removing abnormal protein according to any one of (1) to (4) or a composition for preventing or repairing UV damage according to (5).
(11) A feed or animal drug for suppressing dull complexion or wrinkles or providing moisture-keeping effect, containing a composition for removing abnormal protein according to any one of (1) to (4) or a composition for preventing or repairing UV damage according to (5).
(12) An anti-aging cosmetic for pets, containing a composition for removing abnormal protein according to any one of (1) to (4) or a composition for preventing or repairing UV damage according to (5).
(13) A cosmetic for pets for suppressing dull complexion or wrinkles or providing moisture-keeping effect, containing a composition for removing abnormal protein according to any one of (1) to (4) or a composition for preventing or repairing UV damage according to (5).

### Effects of the Invention

By using highly pure soyasaponin B group as an effective ingredient for removing abnormal protein, effects greater than those provided by conventional soyasaponin can be achieved and a reference quantity not excessive as a normal intake can be set.
By using a composition for removing abnormal protein conforming to the present invention, accumulated abnormal protein can be removed. In other words, formulations conforming to the present invention are effective in preventing or treating diseases and disorders caused by proteolysis abnormality (Alzheimer's disease, Parkinson's disease, dementia with Lewy bodies, triplet repeat disease, amyotrophic lateral sclerosis, cataract, arteriosclerosis, diabetic nephropathy, skin aging due to light, wrinkles, etc.). These formulations are also useful in cosmetics and foods.
To be specific, they are expected to prevent aging, suppress dull complexion and wrinkles, retain moisture, and prevent or repair UV damage.
Specific forms of application include pharmaceuticals, foods, cosmetics and feeds.

### BRIEF DESCRIPTION OF THE DRAWINGS

[FIG. 1] A graph showing the relationship between the administered quantity of saponin and the moisture content in the skin, as measured in a UV irradiation test.
[FIG. 2] A graph showing the relationship between the administered quantity of saponin and the resilience of the skin, as measured in a UV irradiation test.
[FIG. 3] A graph showing the relationship between the administered quantity of saponin and the presence of carbonylated protein in the skin, as measured in a UV irradiation test.
[FIG. 4] A graph showing the relationship between the administered quantity of saponin and the presence of carbonylated protein in the skin, as measured in a paraquat-stressed test.
[FIG. 5] A graph showing the relationship between the administered quantity of saponin and the presence of carbonylated protein in the liver, as measured in a paraquat-stressed test.
[FIG. 6] A graph showing the relationship between the administered quantity of saponin and the presence of carbonylated protein in the red blood cells, as measured in a paraquat-stressed test.

### DETAILED DESCRIPTION OF THE PREFERRED EMBODIMENT

### Best Mode for Carrying Out the Invention

The present invention is explained in details below.
In the present invention, abnormal protein generally refers to protein that has been oxidized, saccharized, or modified by aldehyde, with advancing age.
Soybean-derived saponin, or soyasaponin, is widely found in the seed coat, seed leaf or hypocotyl of the soybean seed or in the leaf, stalk or root of the soybean plant, among others. Soyasaponin has a structure similar to that of glycyrrhizin, but it has a sugar chain consisting of two to five sugars in the triterpenoid structure. Soyasaponin is classified into four groups (A, B, E and DDMP) according to the structure of aglycon (non-sugar part), and saponin in all groups can take various sugar chain structures. To date, eight types of A group, two types of E group, five types of B group and six types of DDMP group have been identified, where aglycon is soyasapogenol A, B, E or DDMP. (Non-Patent Literature: Agric Biol Chem, 55, pp. 315-322 (1991)) (Non-Patent Literature: Agric Biol Chem, 55, pp. 911-917 (1991)) (Non-Patent Literature: Agric Biol Chem, 57, pp. 546-550 (1993)).

A-group saponin is a bisdesmoside saponin with sugar chains bonded to two positions of C-3 and C-22 of soyasapogenol A. It is found only in the soybean seed hypocotyl, and is a main cause of unpleasant taste (bitterness and pungency). Currently A-group saponin is called by two different names: soyasaponin A1 and soyasaponin Ab. Both are the same compound, and similarly A2 and Af, A3 and Ah, A4 and Aa, A5 and Ae, and A6 and Ag, are the same respectively. Ac and Ad have no other name (Non-Patent Literature: BBB, 62 (12), pp. 2291-2299, 1998).

In the meantime, B-group saponin and E-group saponin are both a monodesmoside saponin having only one sugar chain bonded to the C-3 position. DDMP-group saponin can be refined by extracting under mild conditions (Non-Patent Literature: Agric Biol Chem, 57, pp. 546-550 (1993)), but it is converted to B-group saponin under heated or alkali conditions. Therefore, a majority of saponin present in the soybean is either A-group saponin or DDMP-group saponin, and the content of B-group saponin is minimal. It is assumed that a majority of B-group saponin is artifact produced from DDMP-group saponin during the extraction process. B-group saponin is found in abundance in the soybean hypocotyl and seed leaf, and currently B-group saponin is also called by two names: soyasaponin I and soyasaponin Bb. Both are the same compound, and similarly II and Bc, III and Bb', IV and Bc', and V and Ba, are the same, respectively (Non-Patent Literature: BBB, 62 (12), pp. 2291-2299, 1998).

Although the absorption action of soyasaponin is yet to be understood to date, it is assumed that soyasaponin I is not absorbed directly in the human intestines, but instead it is broken down by inner intestinal bacteria into soyasapogenol B, which is a form of aglycon, and then absorbed in a manner dependent upon concentration. It is also suggested that other forms of B-group saponin are also absorbed as forms of aglycon (Non-Patent Literature: J Nutr, 134, pp. 1687-1873 (2004)). Therefore, when B-group saponin is taken through food it is assumed to be absorbed similarly as forms of aglycon regardless of the composition of saponin (glycoside, aglycon and/or derivative thereof, etc.).

In general, many varieties of saponin exhibit hemolytic property. However, soyasaponin has been reported to have virtually no hemolytic property (Non-Patent Literature: Kiso to Rinsho (The Clinical Report), Vol. 15, No. 5, 1981). The inventors measured the hemolytic index of soyasaponin obtained from soybeans in a 2% rabbit blood suspension solution, and found that the hemolytic index was 100 or below as is the case with carrot saponin and that soyasaponin did not have hemolytic property as claimed by other reports.
The inventors also tested soyasaponin B group against mutagenicity and acute toxicity to investigate its safety. As a result, soyasaponin B group exhibited neither and thereby demonstrated high safety.
High-content soyasaponin B group used in the present invention can be obtained using the process explained below.

### [Saponin Extraction]

Material soybean hypocotyl may or may not be delipidized with an organic solvent, etc., in advance, but it is better to delipidize the material as it will increase the saponin extraction efficiency. A general way to extract saponin from material soybean hypocotyl is to add an extraction solvent of 5 to 10 times the volume of material in a temperature ranging from room temperature and 80°C, and then agitate the mixture. However, any other method may be used as long as a sufficient amount of saponin can be extracted.
The ion exchange resin used in the present invention is not limited in any way, as long as it is a weak base anionic exchange resin containing tertiary amine. While a resin having varying grain sizes, such as DIAION WA-30 by Mitsubishi Chemical, can be used, it is preferable to use a resin having uniform grain sizes with 90% or more of grains falling within a range of ±10% of the average grain size.

### [Saponin Elution and Refining]

The solvent is removed from the aforementioned saponin extract by means of distillation and a water-diluted saponin solution is adsorbed to the aforementioned ion exchange resin, after which the resin is washed with water, alcohol or hydrated alcohol to cause the resin-adsorbed saponin to elute using an acid or alkali. A coarsely refined soyasaponin obtained by directly drying the eluted saponin solution has a low purity of 20 to 50 percent by weight and also contains many impurities. The content of soyasaponin B group offering high bioactivity is also low, at around 20 to 30 percent by weight.
To increase the purity of saponin, the eluted saponin is directly diluted with water and then a non-polar synthetic adsorbent is used to adsorb saponin. The non-polar synthetic adsorbent used for this purpose may be styrene-divinyl benzene resin, among others, where examples of such resin include DIAION HP-20 by Mitsubishi Chemical and Amberlite XAD-2 by Rohm and Haas. The alcohol content of the hydrated alcohol used to adsorb saponin to the synthetic adsorbent varies depending on the type of alcohol, but it should preferably be in a range of 0 to 50 percent by weight in the case of methanol, or in a range of 0 to 30 percent by weight in the case of ethanol. Next, the resin is washed with water or hydrated ethanol and then saponin is caused to elute using a different type of hydrated alcohol with a higher alcohol content than the alcohol used for washing the resin, in order to obtain a highly pure saponin solution.

### [Post Processing]

If necessary, the obtained highly pure saponin solution may be pH-adjusted using a pH adjustment agent and then dried by heating and drying, heating and drying under reduced pressure, spray-drying, freeze-drying, etc., to obtain highly pure soyasaponin powder.
The aforementioned process produces highly pure soyasaponin with a purity of 70 percent by weight or higher in an efficient and cost-effective manner. Furthermore, soyasaponin B group offering higher activity can be condensed to a concentration of 50 percent by weight or higher, or the percentage of soyasaponin B group in the total saponin volume can be increased to 70 percent by weight or higher.

The steps of resin-based refinement are explained. After the material is processed with an anionic exchange resin, a non-polar synthetic adsorbent is used to refine saponin. If a synthetic adsorbent is used prior to the anionic exchange resin process, low-polarity substances will be adsorbed strongly to the synthetic adsorbent and the resin will degrade even after a normal resin regeneration process using an alkali, etc., thereby resulting in gradual decrease in the capability of the resin. However, by coarsely refining soyasaponin using an anion exchange resin beforehand, soybean isoflavons, oligosaccharides and low-polarity substances can be removed and the processing capability of the non-polar synthetic adsorbent used in the subsequent process will improve, with the resin not undergoing degradation easily. In this case, the anionic exchange resin can be used repeatedly many times over after alkali processing.

A composition containing a large amount of soyasaponin B group has been confirmed to provide the excellent effects of removing abnormal protein, preventing or repairing UV damage, preventing aging, suppressing dull complexion and wrinkles, and providing moisture-keeping effect, as expected.
These effects can be utilized in pharmaceuticals, foods, drinks, supplements, food additives, cosmetics, feeds, feed additives and animal drugs, among others. They can be provided in forms administrable orally or parenterally.
Highly pure soyasaponin B group can be used directly in various applications, but it can also be mixed with various other components in advance or added to a food, feed or animal drug according to the intended purpose.

Highly pure soyasaponin B group can be used in foods directly, or after mixing with various nutrients. For example, it can be mixed with starch, milk sugar, malt sugar, vegetable oil powder, cacao powder oil, stearic acid or other appropriate auxiliary and then shaped into an edible form, such as granule, pellet, tablet, capsule or paste using a conventional method for use as a health supplement or functional health food. It can also be added to various foods, including processed meat products such as hams and sausages, processed seafood products such as various types of fishcakes, bread, confectionary, butter, powder milk, and fermented milk products. Or, it can also be added to drinks such as water, fruit juice, milk and soda. These agents and foods can be produced by normally used formulation technologies.

Highly pure soyasaponin B group can also be used in cosmetics, where it is added directly or mixed with wheat germ oil or olive oil to obtain a cosmetic ingredient, which will then be used in the production of various cosmetics.

In pharmaceutical applications, highly pure soyasaponin B group can be used in oral or parenteral pharmaceuticals. They are administered by means of mixing the effective ingredient with a solid or liquid nontoxic medical carrier appropriate for the intended administration method such as oral ingestion, intra-rectal administration or injection, to be made into a conventional form of pharmaceutical formulation.

Compositions for parenteral administration may be applied in various forms such as water solution, oil solution, milky liquid, suspension and other liquid forms; gel, cream and other semi-solid forms; and powder, granule, capsule, microcapsule and other solid forms. Highly pure soyasaponin B group can be prepared into any of the aforementioned forms using a known method, to be used in various dosage forms such as lotion, emulsion, gel, cream, ointment, plaster, poultice, aerosol, suppository, injection, powder, etc. These can be applied to the body by means of spreading, attachment or atomization, among others. In particular, lotion, emulsion, cream, ointment, plaster, poultice and aerosol are suitable for external skin medicines. For cosmetic uses, highly pure soyasaponin B group can be used in a cosmetic lotion, milky lotion, cream, mask and other skin care products; makeup base lotion, makeup cream, liquid or cream or pancake foundation, lipstick, eye color, cheek color and other makeup products; or hand cream, leg cream, body lotion and other body care products.

For convenience of use, highly pure soyasaponin B group should be provided in the form of a composition mixed with an extender. Suitable extenders include glucose, lactose, maltose, sucrose and other sugars; sorbitol and other sugar alcohols; dextrin, cyclodextrin and other processed starches; wheat starch, corn starch and other starches; casein, soybean protein and other protein; Arabian gum, sodium alginate, sodium caseinate, gelatin, pectin, powder cellulose, carboxy methyl cellulose and other polymer stabilizers; lecithin, sucrose fatty acid ester, propylene glycol fatty acid ester, glycerin fatty acid ester and other emulsions; and calcium powder, among other.

A composition for removing abnormal protein as proposed by the present invention may contain, in addition to the aforementioned highly pure soyasaponin B group, a compound having an anti-oxidative effect. This compound having an anti-oxidative effect is not specifically limited, and examples include various vitamins; silymarin and various polyphenols; and tocotrienol, coenzyme Q10 and natural components containing the foregoing.
A composition for removing abnormal protein as proposed by the present invention may contain, in addition to the aforementioned highly pure soyasaponin B group, a compound having an effect of promoting biocollagen production. This compound having an effect of promoting biocollagen production is not specifically limited, and examples include collagen and gelatin decomposition products, and peptide mixtures containing a tripeptide having glycine at the N end.

Collagen extracted from the skins, bones and connective tissues such as tendons of animals such as cow, pig and fish, gelatin thermally modified from collagen, and all other forms of collagen can be used. As collagen and/or gelatin decomposition products, it is preferable to use polypeptide containing those with a molecular weight of 400 or below. Polypeptide containing those with an average molecular weight of around 200 to 300 by a large amount is more preferable. Polypeptide containing those with a molecular weight of 400 or below, or more preferably polypeptide containing those with an average molecular weight of around 200 to 300 by a large amount, contains amino acids by a molecular weight of around 100, and is therefore equivalent to polypeptide containing a large amount of tripeptide. This collagen and/or gelatin decomposition product with a molecular weight of 400 or below may have been refined, but refining is not necessary. For example, a mixture with other collagen and/or gelatin decomposition product, etc., may also be used.
On the other hand, a collagen and/or gelatin decomposition product that contains peptide by approx. 400 or below in molecular weight as a specific effective ingredient can contribute to the promotion of collagen production in the body by means of the hydrolysis action of peptide.

Silymarin (CAS No. 65666-07-1) is a general term referring to flavonolignans extracted from silybum marianum (other names: Lady's thistle, milk thistle, CAS No. 84604-20-6). Its confirmed key ingredients include silybin (CAS No. 22888-70-6), silydianin (CAS No. 29782-68-1), silychristin (CAS No. 33889-69-9) and isosilybin (CAS No. 72581-71-6), among others (Tennen Yakubutsu Jiten (Encyclopedia of Natural Medicines) edited by Takuo Okuda). In Europe, silymarin has been used for many years in the prevention and treatment of liver diseases. It is also widely known as an antioxidant. It is a composition useful for skin, and can be used as a formulation for treating psoriasis and atopic skin inflammation (Patent Literature: Japanese Patent Laid-open No. Hei 5-286864), as a composition containing an active complex of flavonolignan and phosphatide and useful in treating exfoliative dermatitis, burns, skin or membrane dystrophy, skin inflammation, preventing skin aging, and protecting the skin from external irritations caused by radiation, wind, sun, etc. (Patent Literature: Japanese Patent No. 2948818), as an agent that permeates into the skin to enhance the natural barrier property of the skin (Patent Literature: Japanese Patent Laid-open No.2000-169328), or as an-agent to suppress sebum secretion (Patent Literature: Japanese Patent Laid-open No. 2000-169332), among others. Normally, silymarin is sold as an extract, which is obtained as dry powder produced by spray-drying an ethanol extracted from silybum marianum seeds. Commercially available silymarin products may be used directly in the present invention. Also, a compound comprising silymarin ingredients, such as silybin, silydianin, silychristin and isosilybin, as isolated from silybum marianum and/or refined, may be used.

A composition for removing abnormal protein as proposed by the present invention may be used in anti-aging or anti-UV applications. Also, a composition containing a compound having an effect of removing abnormal protein, and another compound having an anti-oxidative effect or compound having an effect of promoting biocollagen production, provides an anti-aging composition having an anti-aging effect as well as a function to prevent accumulation of abnormal protein or remove abnormal protein.

A compound having an effect of removing abnormal protein can be used in cosmetics, and the resulting cosmetics can be used for such purposes as preventing aging, suppressing dull complexion and wrinkles, and providing moisture-keeping effect. A composition conforming to the present invention may be taken orally, or injected if the composition has no hemolytic property, or administered parenterally. If taken orally, the composition may be shaped into a form suitable as health food or beauty supplement.

A composition conforming to the present invention may be applied in various forms such as water solution, oil solution, milky liquid, suspension and other liquid forms; gel, cream and other semi-solid forms; and powder, granule, tablets, capsule and other solid forms. A composition conforming to the present invention can be prepared into any of the aforementioned forms using a known method, to be used in various dosage forms such as lotion, emulsion, gel, cream, ointment, plaster, poultice, aerosol, suppository, injection, powder, etc., which are suitable for external skin medicines.

A composition conforming to the present invention may contain vegetable oils and other oils, higher fatty acids, higher alcohols, silicones, anionic surface active agents, cationic surface active agents, ampholytic surface active agents, nonionic surface active agents, preservatives, sugars, metal ion blockers, water-soluble and other polymers, viscosity increasing agents, powders, UV absorbents, UV blockers, hyaluronic acid and other moisture-keeping agents, aromatics, and pH adjustment agents, among others. It can also contain other medicinal components and bioactive components such as vitamins, skin activation agents, blood-circulation promoting agents, normal-bacteria controlling agents, active-oxygen removing agents, anti-inflammatory agents, anticancer agents, whitening agents, and bactericidal agents, among others.

For cosmetic uses, a composition conforming to the present invention can be used in a cosmetic lotion, milky lotion, cream, mask and other skin care products; makeup base lotion, makeup cream, or liquid or cream or pancake foundation; hand cream, leg cream, body lotion and other body care products; bath agents; or hair care products. The composition can be produced in any of these dosage forms according to a formulation method normally used in the production of cosmetics. It can also be used as a lipstick, eye color, cheek color and other makeup products.

If necessary, a composition for removing abnormal protein as proposed by the present invention may contain, in addition to highly pure soyasaponin B group as a key ingredient, a diluent, carrier or any other additive permitted pharmacologically. If necessary a composition conforming to the present invention may also contain any other medicinal compound having pharmacological activity.

Currently, accumulated abnormal protein is associated with certain diseases such as Alzheimer's disease, Parkinson's disease, dementia with Lewy bodies, triplet repeat disease, amyotrophic lateral sclerosis, cataract, arteriosclerosis, diabetic nephropathy, skin aging due to light, dull complexion or wrinkles, and so on. Accordingly, taking a composition for removing abnormal protein as proposed by the present invention will likely enable prevention or treatment of the aforementioned diseases (Non-patent Literature: The FASEB Journal, Vol. 9, pp. 1173-1182, 1995).

A composition conforming to the present invention is useful as a cosmetic or health food to prevent/suppress aging, anti-aging cosmetic or beauty supplement, or cosmetic or health hood to prevent/suppress oxidation. A composition for removing abnormal protein as proposed by the present invention exhibits excellent effects in mammals and is also very safe.

A composition conforming to the present invention is useful in preventing or repairing UV damage to biological tissue, especially skin, which will be or has been exposed to UV ray. The modified protein (abnormal protein) that has been produced in cells due to active oxygen generated by UV exposure can be efficiently broken down to suppress cell damage caused by UV exposure.

Also, the following items may be added according to the specific application or dosage form.
Examples of oils include camellia oil, evening primrose oil, macadamia nut oil, olive oil, rape seed oil, corn oil, sesame oil, jojoba oil, germ oil, wheat germ oil, glycerin trioctanate and other liquid oils; cacao oil, coconut oil, hardened coconut oil, palm oil, palm kernel oil, haze wax oil, haze wax kernel oil, hardened oil, hardened castor oil and other solid oils; and bees wax, candelilla wax, cotton wax, bran wax, lanolin, lanolin acetate, liquid lanolin, sugar cane wax and other waxes.

Examples of hydrocarbons include liquid paraffin, squalene, squalane, and microcrystalline wax.

Examples of higher fatty acids include lauric acid, myristic acid, palmitylic acid, stearic acid, oleic acid, linolic acid, linolenic acid, docosahexaenoic acid (DHA), and eicosapentaenoic acid (EPA).

Examples of higher alcohols include lauryl alcohol, stearyl alcohol, cetyl alcohol, cetostearyl alcohol and other linear alcohols; and monostearyl glycerin ether, lanolin alcohol, cholesterol, phytosterol, octyl dodecanol and other branched alcohols.

Examples of silicones include dimethyl polysiloxane and methyl phenyl polysiloxane based on chained polysiloxane, as well as decamethyl cyclopentane siloxane based on cyclic polysiloxane.

Examples of anionic surface active agents include sodium laurate and other fatty acid salts, sodium lauryl sulfate and other higher alkyl sulfuric ester salts, POE lauryl sulfate triethanolamine and other alkyl ether sulfuric ester salts, N-acyl sarcosinic acid, sulfosuccinic acid salt, and N-acyl amino acid salt.

Examples of cationic surface active agents include stearyl trimethyl ammonium chloride and other alkyl trimethyl ammonium salts, benzalkonium chloride, and benzethonium chloride.

Examples of ampholytic surface active agents include alkyl betaine, amide betaine and other betaine surface active agents.

Examples of nonionic surface active agents include sorbitan monooleate and other sorbitan fatty acid esters, and hardened caster oil derivatives.

Examples of preservatives include methyl paraben and ethyl paraben.
Examples of metal ion blockers include ethylene diamine tetraacetic disodium, edetic acid, sodium edetate and other edetates.

Examples of polymers include Arabian gum, tragacanth gum, galactan, guar gum, carageenan, pectin, agar, quince seed, dextran, pullulan, carboxy methyl starch, collagen, casein, gelatin, methyl cellulose, methyl hydroxy propyl cellulose, hydroxy ethyl cellulose, sodium carboxy methyl cellulose (CMC), sodium alginate, carboxy vinyl polymer (CARBOPOL, etc.) and other vinyl polymers.

Examples of viscosity increasing agents include carageenan, tragacanth gum, quince seed, casein, dextrin, gelatin, CMC, hydroxy ethyl cellulose, hydroxy propyl cellulose, carboxy vinyl polymer, guar gum, xanthan gum, and bentonite.

Examples of powders include talc, kaolin, mica, silica, zeolite, polyethylene powder, polystyrene powder, cellulose powder, inorganic white pigment, inorganic red pigment, titanium oxide coated mica, titanium oxide coated talc, colored titanium oxide coated mica and other pearl pigments, and organic pigments such as red 201 and red 202.

Examples of UV absorbents include para-aminobenzoic acid, phenyl salicylate, para-methoxy isopropyl cinnamate, para-methoxy octyl cinnamate, and 2,4-dihydroxy benzophenone.

Examples of UV blockers include titanium oxide, talc, carmine, bentonite, kaolin, and zinc oxide.

Examples of moisture-keeping agents include polyethylene glycol, propylene glycol, dipropylene glycol, 1,3-butylene glycol, 1,2-pentane diol, glycerin, diglycerin, polygrycerin, xylitol, maltitol, maltose, sorbitol, dextrose, fructose, chondroitin sodium sulfate, sodium hyaluronate, sodium lactate, pyrrolidone carboxylic acid, and cyclodextrin.

Examples of medicinal components include vitamins such as vitamin A oil, retinol and other forms of vitamin A; riboflavine and other forms of vitamin B₂; pyridoxine hydrochloride and other forms of vitamin B₆; L-aseorbic acid, L-ascorbic acid ester phosphate, L-ascorbic acid ester monopalmitate, L-ascorbic acid ester dipalmitate, L-ascorbic acid-2-glucoside and other forms of vitamin C; calcium pantothenate and other pantothenic acids; vitamin D₂, cholecalciferol and other forms of vitamin D; and α-tocopherol, tocopherol acetate, DL-α-tocopherol nicotinate and other forms of vitamin E.

Placenta extract, glutathione, saxifraga stolonifera extract and other whitening agents; royal jelly, beech extract and other skin activating agents; capsaicine, zingherone, cantharidis tincture, ichthammol, caffeine, tannic acid, γ-oryzanol and other blood-circulation promoting agents; glycyrrhizinic acid derivative, glycyrrhetinic acid derivative, azulene and other anti-inflammatory agents; arginine, serine, leucine, tryptophane and other amino acids; and normal-bacteria controlling agents such as maltose sucrose condensate and lysozyme chloride are included.

Chamomile extract, parsley extract, beech extract, wine yeast extract, grapefruit extract, Japanese honeysuckle extract, rice extract, grape extract, hop extract, rice bran extract, Japanese mandolin extract, cork tree bark extract, coix seed extract, swertia japonica extract, melilot extract, birch extract, liquorice extract, peony extract, soapwort extract, dishcloth gourd extract, capsicum extract, lemon extract, gentian root extract, perilla frutescens extract, aloe extract, rosemary extract, sage extract, thyme extract, tea extract, seaweed extract, cucumber extract, clove extract, carrot extract, marronnier extract, hamamelis extract, mulberry extract and various other extracts are also included.

The present invention is explained in further details below using examples. It should be noted, however, that the present invention is not limited to these examples.

### Example 1

Soyasaponin was fed to hairless mice for 70 days, after which they were exposed to UVA and UVB to examine the utility of highly pure soyasaponin B group.

### [Test of effects of highly pure soyasaponin B group on the skin of UV-irradiated mice]

The test was conducted under the following conditions using hairless mice (Hos: HR-1, male) of six weeks old at the start of test.

### 1) Preparation and administration of tested items

On the first day of administration, hairless mice in good general condition were divided into groups by weight so that each group contained five mice and there was not much difference between adjacent groups. Mice were raised using one cage for each group. Crude soyasaponin (14.9% of soyasaponin A group and 25.8% of soyasaponin B group) was suspended in distilled water to a total saponin volume of 50 mg/kg per body weight ((a) crude material, 123 mg/kg), while soyasaponin B group (9.5% of soyasaponin A group and 69.4% of soyasaponin B group) was suspended in distilled water to a soyasaponin B group volume of 50 mg/kg per body weight ((b) saponin B material, 72 mg/kg). Both were fed once a day for 10 weeks by means of forced oral administration using a stomach tube. On days the mice were irradiated with UV, administration occurred two hours after the irradiation. The rearing conditions are shown in Table 1.

**[Table 1]**

| Group name | (a) Administered quantity of crude A material | (b) Administered quantity of saponin B material | UV irradiation |
|---|---|---|---|
| Control | 0 mg/kg | 0 mg/kg | - |
| Crude soyasaponin | 123.0 mg/kg | 0 mg/kg | - |
| Soyasaponin B group | 0 mg/kg | 72.0 mg/kg | - |
| UV irradiation | 0 mg/kg | 0 mg/kg | + |
| UV irradiation + crude soyasaponin | 123.0 mg/kg | 0 mg/kg | + |
| UV irradiation + soyasaponin B group | 0 mg/k | 72.0 mg/kg | + |

### 2) Measurement of moisture content in the skin

Moisture content was measured three times using a moisture checker at a position 2 cm toward the neck from the base of the tail on the back, and 0.5 cm from the lumbar vertebrae to the right, and an average was obtained. Moisture content was measured on the starting day of the test, intermediate observation days, and dissection analysis day.

### 3) Measurement of resilience of the skin

Resilience of the skin was measured three times using a skin-resilience measuring apparatus (Non-patent Literature: Cutometer SEM 575, by Electronic GmbH co.) at a position 2 cm toward the neck from the base of the tail on the back, and 0.5 cm from the lumbar vertebrae to the right, and an average was obtained. Resilience of the skin was measured on the dissection analysis day.

### 4) Measurement of oxidized protein

Oxidized protein, or carbonylated protein, was measured by marking carbonylated protein using 2,4-dinitrophenyl hydrazine (DNPH) that specifically bonds with the carbonic group in protein produced by oxidization disorder, and then detecting DNPH using an anti-DNPH antibody that bonds specifically with DNPH. The specific method is explained below. A piece of skin of 1 cm² in size was cut out from the area irradiated with UV, and homogenized by adding 1 ml of 0.1M tris buffer (pH 7.5). The sample was then centrifugally separated at 10,000 rpm for 15 minutes, and the supernatant was filtered and then analyzed. Bonding of protein and dinitrophenyl hydrazine (DNPH) was implemented using a known method (Non-patent Literature: Nakamura, et al., Journal of Biochemistry, Vol. 119, pp. 768-774, 1996). DNPH-bonded protein was separated by SDS-PAGE and a protein transfer apparatus was used to transfer the separated protein onto a polyvinylidene difluoride (PVDF) membrane. After the transfer the membrane was blocked for at least 2 hours in a PBS (-) solution containing 5% skim milk, after which the membrane was washed and reacted with an anti-DNPH antibody for 2 hours. Thereafter, the membrane was washed and reacted with biotinized anti-rabbit immunoglobulin G for 1 hour. Then, the PVDF membrane was washed and exposed to light using a fluorescent detection kit (ECL PLUS), and an image was transferred using a medical automatic development apparatus. The image was analyzed using a densitometer.

### 5) UV irradiation

Mice were irradiated with UV after having been moved to a dedicated PC cage, where each group was irradiated with 20 mj/cm² of UVB and 14 j/cm² of UVA. Irradiation was carried out for 10 weeks based on three-days-a-week cycles on Mondays, Wednesdays and Fridays.

### 6) Dissection analysis

With each group, mice were given no food for 18 hours from the day immediately following the last day of the test period, and then given Nembutal (40 mg/kg) by means of intra-abdominal administration, after which they were dissected for analysis under anesthesia. The applicable exposure dose and period have been reported to cause wrinkles in skin, just like wrinkles in human skin caused by skin aging due to light (Non-patent Literature: J of Dermatological Science, 27, pp 519-525 (2001)).

### 7) Static processing

The test result was indicated by "average ± standard deviation," and a statistical significance test was conducted according to the Student's t-test using Excel.

### 1. Observation of general condition

From around week 4, UV-irradiated mice began showing slight browning of the skin in the head and formation of deep wrinkles in the neck and back of rear legs more prominently than mice not irradiated with UV.
In the exterior observation at the time of dissection analysis, UV-irradiated mice had clear wrinkles in the face, neck and back of rear legs. However, the conditions were less conspicuous with UV-irradiated mice fed with crude soyasaponin group and soyasaponin B group, and especially mice fed with soyasaponin B group had shallower wrinkles and the color of wrinkles also looked lighter to the naked eyes. Mice fed with soyasaponin B group also had softer skin compared to those in the control group, and this softness of skin was particularly prominent with UV-irradiated mice fed with soyasaponin B group.

### 2. Weight change

There was no significant difference in weight change among the groups.

### 3. Feed intake

As with weight change, there was no significant difference in feed intake.

### 4. Liver weight

There was no significant difference in liver weight, either, and no abnormal conditions were observed. No other organ exhibited any abnormality.

### 5. Moisture content in the skin

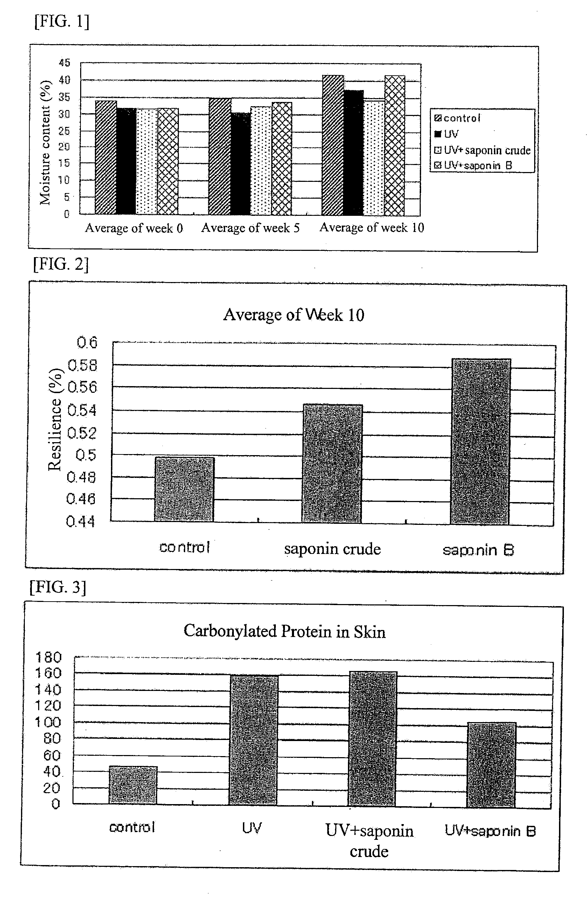

It is assumed that the higher the measured value, the more the skin stores water and the greater moisture-keeping function it demonstrates. It is generally known that UV irradiation reduces the moisture content in the skin. In this test, too, UV-irradiated mice showed lower moisture contents than those in the control group on the intermediate observation days and dissection analysis day. On the intermediate observation days, both groups of UV-irradiated mice fed with soyasaponin showed higher moisture contents than UV-irradiated mice not fed with soyasaponin. On the dissection analysis day, UV-irradiated mice fed with crude soyasaponin showed lower moisture contents than UV-irradiated mice not fed with soyasaponin, while UV-irradiated mice fed with soyasaponin B group showed a higher average equivalent to the moisture content of the control group. The results are shown in FIG. 1.

### 6. Resilience of the skin

It is assumed that the higher the measured value, the more resilient the skin is. It is generally known that UV irradiation reduces the resilience of the skin. The results are shown in FIG. 2. Among mice not irradiated with UV, those fed with soyasaponin B group had significantly higher resilience than others fed with crude soyasaponin.

### 7. Weight of oxidized protein

The results are shown in FIG. 3. Weight of oxidized protein is detected as weight of carbonylated protein. The smaller the weight of carbonylated protein, the smaller the amount of protein accumulated in the organs. As evident from FIG. 3, the weight of oxidized protein increased significantly in UV-irradiated mice compared to non-UV-irradiated mice. However, UV-irradiated mice that were also fed with soyasaponin B group showed a significant reduction in the weight of oxidized protein, demonstrating the effect of removing oxidized protein provided by highly pure soyasaponin B group.
In particular, while mice fed with crude soyasaponin showed a weight of oxidized protein equivalent to that of UV-irradiated mice not fed with soyasaponin, mice fed with soyasaponin B group showed a clearly lower level, suggesting the action of soyasaponin B.

From the above, it has been confirmed that highly pure soyasaponin B group suppresses the reduction of moisture content and prevents dryness and wrinkle generation caused by skin-stressing UV irradiation, based on comparison with mice fed with normal crude soyasaponin and those in the control group. Also, mice fed with highly pure soyasaponin B group reduced the weight of oxidized protein compared to those fed with crude saponin and in the control group, indicating the effectiveness of highly pure soyasaponin B group in removing oxidized protein.
It was also revealed that administration of highly pure soyasaponin B group would enhance the resilience of the skin. Administering 50 mg/kg of highly pure soyasaponin B group to a mouse is equivalent to a person weighing 60 kg taking approx. 450 mg per day based on the body surface area conversion formula of y = (³√x)², and this is a quantity that can be easily taken through food.

### Example 2

### [Test of effects of highly pure soyasaponin B group on paraquat-stressed rats]

The test was conducted under the following conditions using rats (Wistar/Cri, male) of six weeks old at the start of test.

### 1) Preparation and administration of tested items

On the first day of administration, Wistar rats in good general condition were divided into groups by weight so that each group contained five rats and there was not much difference between adjacent groups. Individual rats were raised separately. Crude soyasaponin (14.9% of soyasaponin A group and 25.8% of soyasaponin B group) was given as a mixed feed, where it was mixed with MF feed by Oriental Yeast to a total saponin intake of 15 mg/kg per body weight. Soyasaponin B group (9.5% of soyasaponin A group and 69.4% of soyasaponin B group) was also given as a mixed feed, where it was mixed with MF feed by Oriental Yeast to an intake of soyasaponin B group of 15 mg/kg per body weight. As for paraquat administration, 1,000 mg of paraquat was dissolved in 500 ml of saline solution and 10 mg/kg per body weight of the prepared injection solution was injected into the abdominal cavity of the rat once a week for 2 months. The rearing conditions are shown in Table 2.

**[Table 2]**

| Group name | Administered quantity of saponin | Administration of paraquat |
|---|---|---|
| Control | 0 mg/kg | - |
| Paraquat | 0 mg/kg | + |
| Paraquat + small amount of soyasaponin B group | 15 mg/kg | + |
| Paraquat + crude soyasaponin | 15 mg/kg | + |

### 2) Dissection analysis

With each group, rats were given no food for 18 hours from the day immediately following the last day of the test period, and then given Nembutal (40 mg/kg) by means of intra-abdominal administration, after which they were dissected for analysis under anesthesia.

### 3) Statistical processing

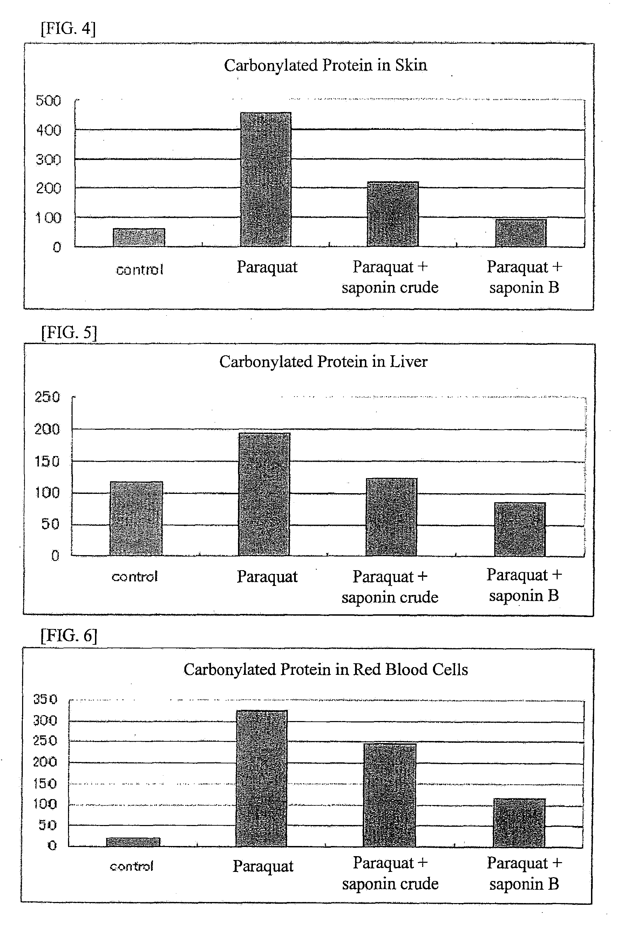

The test results are shown in FIGS. 4 through 6.

### 1. Observation of general condition

Since paraquat is a strong pesticide that may even cause acute poisoning, rats normally exhibit lower body temperatures, loss of vitality and softening of feces immediately after the administration of paraquat. However, paraquat-stressed rats fed with soyasaponin exhibited these conditions to a lesser degree and recovered to the same state of health as the control group two days after the administration. In particular, paraquat-stressed rats fed with soyasaponin B group exhibited only minor symptoms, and this trend remained the same in the latter half of the test period.

### 2. Weight change

There was no significant difference in weight change among the groups.

### 3. Feed intake

As with weight change, there was no significant difference in feed intake.

### 4. Measurement of weight of oxidized protein (skin, red blood cells, liver)

Each collected sample (1 cm² of skin, 100 µl of blood or 100 mg of liver) was mixed with 1 ml of 0.1M tris buffer (pH 7.5) and homogenized. The sample was then centrifugally separated at 10,000 rpm for 15 minutes, and the supernatant was filtered and then analyzed. Bonding of protein and dinitrophenyl hydrazine (DNPH) was implemented using a known method (Non-patent Literature: Nakamura, et al., Journal of Biochemistry, Vol. 119, pp. 768-774, 1996). DNPH-bonded protein was separated by SDS-PAGE and a protein transfer apparatus was used to transfer the separated protein onto a polyvinylidene difluoride (PVDF) membrane. After the transfer the membrane was blocked for at least 2 hours in a PBS (-) solution containing 5% skim milk, after which the membrane was washed and reacted with an anti-DNPH antibody for 2 hours. Thereafter, the membrane was washed and reacted with biotinized anti-rabbit immunoglobulin G for 1 hour. Then, the PVDF membrane was washed and exposed to light using a fluorescent detection kit (ECL PLUS), and an image was transferred using a medical automatic development apparatus. The image was analyzed using a densitometer.

The results are shown in FIGS. 4 through 6. Weight of oxidized protein is detected as weight of carbonylated protein. As evident from FIGS. 4 through 6, the weight of oxidized protein increased significantly in paraquat-stressed rats, but the increase was significantly lower in paraquat-stressed rats fed with highly pure soyasaponin B group. This difference was greater than what was observed with paraquat-stressed rats fed with crude soyasaponin. These results demonstrate the effect of removing oxidized protein provided by highly pure soyasaponin B group.

The above results show that highly pure soyasaponin B significantly reduced the weight of oxidized protein in the skin, liver and red blood cells of paraquat-stressed rats, based on comparison with rats fed with normal crude soyasaponin and those in the control group. Administering 15 mg/kg of highly pure soyasaponin B group to a rat is equivalent to a person weighing 60 kg taking approx. 134 mg per day based on the body surface area conversion formula of y = (³√x)², and this is a quantity that can be easily taken through food.

Formulation Example 1
[Capsule]
Composition
Soyasaponin (soyasaponin B content 50%) --- 50 mg
Tocotrienol --- 30 mg
Bees wax ---10 mg
Grape seed oil --- 110 mg
The above ingredients were mixed and then filled into base capsules to which gelatin and glycerin were added, to obtain soft capsules.

Formulation Example 2
[Tablet]
Composition
Soyasaponin (soyasaponin B content 50%) --- 25 mg
Silybum marianum (silymarin content 65%) --- 20 mg
Collagen hydrolyzate --- 50 mg
Cellulose --- 40 mg
Starch --- 20 mg
Sucrose fatty acid ester --- 2 mg
The above ingredients were mixed, hardened, and then stamped into a tablet shape to obtain tablets.

Formulation Example 3
[Juice]

| (Composition) | (Content: percent by weight) |
|---|---|
| Fructose/dextrose solution | 5.00 |
| Citric acid | 10.4 |
| L-ascorbic acid | 0.20 |
| Aromatic agent | 0.02 |
| Colorant | 0.10 |
| Gelatin decomposition product (average molecular weight 300) | 1.00 |
| Soyasaponin (soyasaponin B content 50%) | 1.00 |
| Water | 82.28 |

Formulation Example 4
[Cream]

| | |
|---|---|
| (1) Stearyl alcohol | 6.0 |
| (2) Stearic acid | 2.0 |
| (3) Hydrogenated lanolin | 4.0 |
| (4) Squalane | 9.0 |
| (5) Octyl dodecanol | 10.0 |
| (6) POE (25) cetyl alcohol ether | 3.0 |
| (7) Glycerin monostearate | 2.0 |
| (8) Gelatin decomposition product (average molecular weight 300) | 1.00 |
| (9) Soyasaponin (soyasaponin B content 50%) | 1.00 |
| (10) Preservative | As appropriate |
| (11) Aromatic agent | As appropriate |
| (12) 1,3-butylene glycol | 6.0 |
| (13) PEG 1500 | 4.0 |
| (14) Purified water | Remainder |

Of the above ingredients, (1) through (11) were heated to 80°C and dissolved to produce an oil phase. Also, ingredients (12) through (14) were heated to 70°C and dissolved to produce a water phase. The water phase was gradually added to the oil phase to cause emulsification, and the mixture was cooled to 40°C under agitation, and then further cooled to 30°C under agitation to obtain cream.

## Claims

1. A composition for removing abnormal protein, **characterized by** having soyasaponin B group as a main ingredient.

2. The composition for removing abnormal protein according to Claim 1, **characterized in that** soyasaponin B group is a soybean extract containing 40 to 100 parts by weight of soyasaponin B group relative to 100 parts by weight of the soybean extract.

3. The composition for removing abnormal protein according to Claim 1 or 2, **characterized in that** soyasaponin B group is soyasaponin I, II, III, IV or V or acetylated form thereof, and soyasaponin A group is soyasaponin A1, A2, A3, A4, A5, A6, Ac or Ad or acetylated form or mixture thereof.

4. The composition for removing abnormal protein according to any one of Claims 1 to 3, **characterized by** containing one or more of collagen, gelatin, collagen hydrolyzate and gelatin hydrolyzate, and/or silymarin.

5. A composition for preventing or repairing UV damage, containing the composition for removing abnormal protein according to any one of Claims 1 to 4.

6. An anti-aging food containing the composition for removing abnormal protein according to any one of Claims 1 to 4 or the composition for preventing or repairing UV damage according to Claim 5.

7. A food for suppressing dull complexion or wrinkles or providing moisture-keeping effect, containing the composition for removing abnormal protein according to any one of Claims 1 to 4 or the composition for preventing or repairing UV damage according to Claim 5.

8. An anti-aging cosmetic containing the composition for removing abnormal protein according to any one of Claims 1 to 4 or the composition for preventing or repairing UV damage according to Claim 5.

9. A cosmetic for suppressing dull complexion or wrinkles or providing moisture-keeping effect, containing the composition for removing abnormal protein according to any one of Claims 1 to 4 or the composition for preventing or repairing UV damage according to Claim 5.

10. An anti-aging feed or animal drug containing the composition for removing abnormal protein according to any one of Claims 1 to 4 or the composition for preventing or repairing UV damage according to Claim 5.

11. A feed or animal drug for suppressing dull complexion or wrinkles or providing moisture-keeping effect, containing the composition for removing abnormal protein according to any one of Claims 1 to 4 or the composition for preventing or repairing UV damage according to Claim 5.

12. An anti-aging cosmetic for pets, containing the composition for removing abnormal protein according to any one of Claims 1 to 4 or the composition for preventing or repairing UV damage according to Claim 5.

13. A cosmetic for pets for suppressing dull complexion or wrinkles or providing moisture-keeping effect, containing the composition for removing abnormal protein according to any one of Claims 1 to 4 or the composition for preventing or repairing UV damage according to Claim 5.
